# EUROPÄISCHE PATENTSCHRIFT

(11) **EP 2 740 412 B1**
(45) Veröffentlichungstag und Bekanntmachung des Hinweises auf die Patenterteilung: **20.01.2016**
(21) Anmeldenummer: 12008240.9
(22) Anmeldetag: 10.12.2012
(51) Int. Cl.: A61B 17/00

(54) **Implantierbares Verschlusssystem mit membranbespanntem Formgedächtnisgeflecht**
Implantable closure system with membrane-covered shape-memory braid
Système d'obturation implantable basé sur une structure tréssée à partir de matière à mémoire de forme et recouverte d'une membrane

(43) Veröffentlichungstag der Anmeldung: 11.06.2014
(73) Patentinhaber: Peter Osypka Stiftung, 79639 Grenzach-Wyhlen (DE)
(72) Erfinder: Osypka, Peter, Prof.Dr.Ing., 79639 Grenzach-Wyhlen (DE)

(56) Entgegenhaltungen:
- EP-A1- 2 340 770
- DE-U1-202010 011 724
- US-A1- 2006 229 670

## Beschreibung

Die Erfindung betrifft ein implantierbares Verschlusssystem mit dem Öffnungen in Organen oder Körperoberflächen verschlossen werden können, wobei das Verschlusssystem zum Verschließen einer Öffnung im Myokard der linken Herzkammerspitze geeignet ist.

Das Verschlusssystem besteht aus einem membranbespannten fein drahtigen Schirmgeflecht mit einem Fixierungselement, wobei die Membran hierbei die zu verschließende Öffnung abdichtet und das Fixierungselement das Verschlusssystem im Gewebe verankert.

Darüber hinaus erlaubt das gleiche System die Einführung und Fixierung von temporären Stimulationselektroden (Herzdrähten).

Chirurgische Operationen werden oft mittels minimalinvasiver Methoden durchgeführt. Hierbei kann auf eine großflächige Öffnung der Körper- oder Organoberfläche verzichtet werden, indem die gesamte Prozedur durch kleine Öffnungen durchgeführt wird. Der Vorteil liegt hier in einer verkürzten Prozedur mit einem deutlich verringerten Infektionsrisiko.

Die für die Operation notwendigen Werkzeuge werden durch sogenannte Trokare (Hilfsinstrumente, welche einen Kanal durch die Körper- oder Organoberfläche darstellen) geführt. Der Ersatz einer Aortenklappe wird seit vielen Jahren durch eine Operation am offenen Herzen unter Einsatz einer Herz-Lungen Maschine durchgeführt. Mit zunehmendem Alter der Patienten erhöht sich das Risiko dieser Methode.

In den letzten Jahren sind weitere Entwicklungen des aortalen Herzklappenersatzes bekannt geworden, wie beispielsweise der perkutane Katheter-gesteuerte Klappenersatz der unter dem Namen TAVI (Transcatheter Aortic Valve Implantation) bekannt ist. Dieser Eingriff ist ohne das Öffnen der Brust und dem Einsatz einer Herz-Lungen-Maschine möglich. Beim TAVI-Verfahren wird die Klappe auf einem Ballon fixiert, der per Katheter in den Körper und zur Implantation ins Herz eingeführt wird. Wenn die Klappe sich innerhalb der defekten Aortenklappe befindet, wird der Ballon aufgedehnt und die Klappe exakt positioniert.

Eine andere Möglichkeit ist die minimal-invasive transapikale Aortenklappen-Implantation am schlagenden Herzen unter Verzicht des Einsatzes der Herz- Lungen-Maschine. Die alte Herzklappe wird dabei nicht wie üblich entfernt, sondern von einer zusammenfaltbaren Klappe, die über einen Katheter durch die Herzspitze eingeführt wird, überdeckt.

Bei der transapikalen Methode wird unterhalb der Herzspitze eine kleine Öffnung chirurgisch vorgenommen. Danach wird ein Trokar durch die Herzwand in den linken Ventrikel des Herzens eingeführt. An der Einstichstelle des Herzens wird chirurgisches Nähmaterial so angeordnet, dass nach der Prozedur, die durch den Einstich-Trokar erfolgte Öffnung wieder zügig verschlossen werden kann. Die Dichtigkeit derartig zugenähter Öffnungen im Myokard, insbesondere beim normalen Arbeitsdruck des Herzens, lässt oft zu wünschen übrig.

So kann es sein, dass die Naht wieder aufreißt.

Das deutsche Gebrauchsmuster DE20 2010 011 724 U1 beschreibt ein implantierbares Verschlusssystem aus einem membranbespannten Schirm und einer Fixierungsvorrichtung. Die Bespannung haftet auf einen relativ groben Nitinolgestänge.

Die US-Patentanmeldung US2006229670 beschreibt ein Verschlusssystem wobei ein Schirmgeflecht in die zu verschließende Körperöffnung eingesetzt wird. Das Abdichten der Körperöffnung erfolgt durch Ausbringen von härtenden Materialien.

Die EP-Patentanmeldung EP2340770 beschreibt einen aus Metallstegen geformten membranbespannten Occluder.

Der Erfindung liegt somit die Aufgabe zugrunde ein Verschlusssystem zu entwickeln, durch welches eine durch den Einstich-Trokar erfolgte Öffnung, insbesondere eine Öffnung im Myokard, zügig und dicht verschlossen werden kann, sodass spätere Blutungen ausgeschlossen sind.

Die Aufgabe wird gelöst durch ein Verschlusssystem bestehend aus:
a) einem elastischen Element (6) das ein distales und ein proximales Ende aufweist;
b) mindestens einem Schirmgeflecht (7) mit einer Außenseite und einer Innenseite, das sich entweder am distalen oder am proximalen Ende des elastischen Elements befindet;
c) einem Fixierungselement (8) das sich entweder am distalen oder am proximalen Ende des elastischen Elements befindet und dem Schirmgeflecht gegenüber angeordnet ist;
wobei das Schirmgeflecht aus Nitinoldraht besteht und mindestens 30 Fäden mit einem Durchmesser von 0.03-0,15mm aufweist und mindestens eine Seite des Schirmgeflechts eine Membranbespannung (70, 71) aufweist

Wesentlich ist, dass das Geflecht weich und flexibel ist, um das sehr weiche Herzgewebe nicht zu schädigen. Das Geflecht ist den anatomischen Verhältnissen der linken Herzkammer angepasst und besteht mindestens aus 30 Fäden, vorzugsweise aus 30-100 Fäden, besonders bevorzugt aus 70 -90 Fäden mit einem Durchmesser von 0,03-0,15mm, vorzugsweise 0,05-0,13mm; besonders bevorzugt circa 0,1 mm.

Das Schirmgeflecht besteht vorzugsweise aus Nitinoldraht und erlaubt somit ein selbstständiges Entfalten der Membran.

Mindestens ein membranbespanntes Schirmgeflechts muss vorhanden sein. Es ist aber auch möglich, dass ein zweites membranbespanntes Schirmgeflecht, welches sich gegenüber dem ersten Schirmgeflecht befindet, eine zusätzliche Abdichtungsmöglichkeit darstellt.

Zur Bespannung des Geflechts eignet sich ein biokompatibler Kunststoff wie z. B. Silikon oder Polyurethan, vorzugsweise Silikon. Wesentlich ist, dass die Membran flüssigkeitsdicht ist, sodass kein Blut austreten kann. Die Membran sorgt unmittelbar nach Applizierung des Schirmgefechts für eine Abdichtung des Myokards.

Die Bespannung befindet sich auf der Außenseite oder auf der Innenseite des Schirmgeflechts, vorzugsweise auf der Außenseite. Denkbar ist auch eine Bespannung auf beiden Seiten.

Das elastische Element ist beispielsweise eine Feder, vorzugsweise eine Schraubenfeder. Es ist aber auch denkbar, dass ein elastischer Kunststoff, wie z.B. Gummi verwendet wird. Die Schraubenfeder ist beispielsweise aus Edelstahl oder aus einer Ni-Co-Legierung wie z.B. MP35N.

Wird eine Schraubenfeder verwendet, so ist es möglich das Ausmaß der Dehnung der Schraube zu begrenzen. Hierzu kann ein flaches und vorzugsweise aus Draht bestehendes Band an die Innenseite der Schraube gekoppelt werden.

Das Fixierungselement ist vorzugsweise eine Hakenstruktur. Es sind aber auch andere Strukturen denkbar wie Stifte, Nägel, Klammern, Schrauben, Spiralen. Wichtig ist, dass sich das vorgespannte Fixierungselement bei Zurückziehen des Trokars entspannt und sich im Myokard verankert. Die Fixierung des Schirmgeflechts an der Öffnung des Myokards wird durch vorgespannte Hakenstrukturen welche sich im umliegenden Herzmuskelgewebe verankern, erzielt.

Die Haken sind vorzugsweise aus Nitinol gefertigt, wobei die Formgebung der Haken variieren kann. Durch unterschiedlich ausgeführte Formen der Hakenstruktur kann die Haltekraft beeinflusst werden.

Vor der Anwendung ist das Verschlusssystem zusammengefaltet und befindet sich im Tubus eines Trokars.

Das Verfahren zum Verschließen der Trokar-Einstichöffnung umfasst die folgenden Schritte:
a) der Trokar (1), welcher das oben beschriebene Verschlusssystem in zusammengeklappter Form enthält, wird durch die Myokardwand geschoben,
b) das Schirmgeflecht (7) wird aus dem Trokar herausgeschoben und entfaltet sich, während das Fixierungselement (8) noch im Trokar verbleibt;
c) durch Zug am elastischem Element (6) wird das Fixierungselement vorgespannt und unterhalb die Myokardöffnung gezogen;
d) nach Zurückziehen des Trokars unter Spannung wird das Fixierungselement entspannt und dadurch im Myokardgewebe verankert.

Abbildungen:
Die Erfindung wird an Hand der Abbildungen näher erläutert.
Fig. 1 zeigt eine typische Darstellung der transapikalen Aorten klappen Implantation (TAAVI).
Fig. 2 zeigt eine beispielhafte Ausführungsform des Verschlusssystems.
Fig. 3 zeigt eine weitere beispielhafte Ausführungsform des Verschlusssystems.
Fig. 4 zeigt eine beispielhafte Ausführungsform des Verschlusssystems in der linken Ventrikel Spitze mit einer zusätzlichen Einführung von temporären Elektroden für eine mögliche externe Herz Stimulation.
Fig. 5a-5d zeigen den Ablauf der Einführung und Fixierung der temporären Elektroden für die Stimulation des Herzens.
Fig. 6 zeigt eine weitere beispielhafte Ausführungsform der temporären Stimulationselektrode.
Fig. 7 a und b zeigt eine Ausführungsform des erfindungsgemässen Verschlusssystems im Detail.
Fig. 8 -10 zeigt beispielhaft das Platzieren des erfindungsgemässen Verschlusssystems.

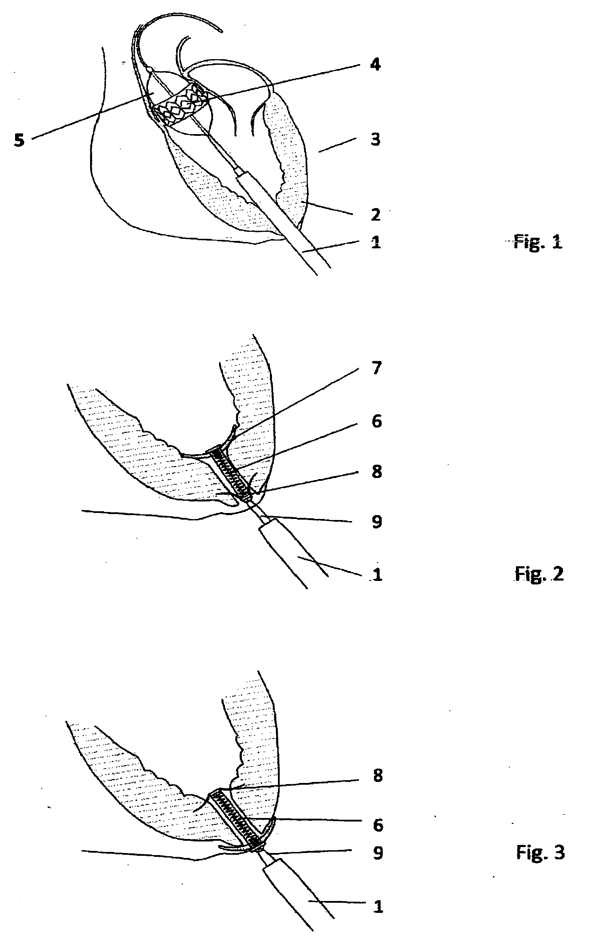

**Fig. 1** zeigt eine typische Darstellung der transapikalen Aortenklappen Implantation (TAAVI). Der Trokar (1) wird durch das Myokard (2) des linken Ventrikels des Herzens (3) in die linke Herzkammer geführt. Durch den Trokar (1) wird die künstliche Herzklappe (4) mit Hilfe eines Ballons (5) aufgedehnt und fixiert. Danach wird der Ballonkatheter wieder entfernt.

**Fig. 2** zeigt eine beispielhafte Ausführungsform des erfindungsgemässen Verschlusssystems das durch einen Trokar (1) eingeführt wurde. Die Trokar-Einstichöffnung ist abgedichtet und fest verschlossen. Das membranbespannte Schirmgeflecht (7) befindet sich an der Innenseite des Myokards am proximalen Ende des elastischen Elements (6). Das hakenförmige Fixierungselement (8) befindet sich am distalen Ende des elastischen Elements (6) und ist über das elastische Element (hier eine Schraubenfeder) mit dem Schirm-geflecht (7) verbunden. Das Schirmgeflecht (7) ist den anatomischen Verhältnissen der linken Herzkammer angepasst und wird mit Hilfe der Schraubenfeder (6) durch die Hakenstruktur (8) im umliegenden Myokardgewebe fixiert. Die Schraubenfeder wird durch Zug am Doppelfaden (9) vorgespannt und unterhalb die Myokardöffnung gezogen. Nach Entspannung und erfolgter Fixierung wird der Faden durch Zug an einer Fadenseite leicht entfernt.

**Fig. 3** zeigt eine weitere beispielshafte Ausführungsform des Verschlusssystems das in der linken Ventrikel-Spitze platziert ist. Sollten die anatomischen Verhältnisse in der Spitze des linken Ventrikels so beschaffen sein, dass eine Abdichtung an der Innenseite des Myokards erschwert ist, kann das Verschlusssystem (6) auch in umgekehrter Weise, das heißt an der Außenseite des Myokards implantiert werden.

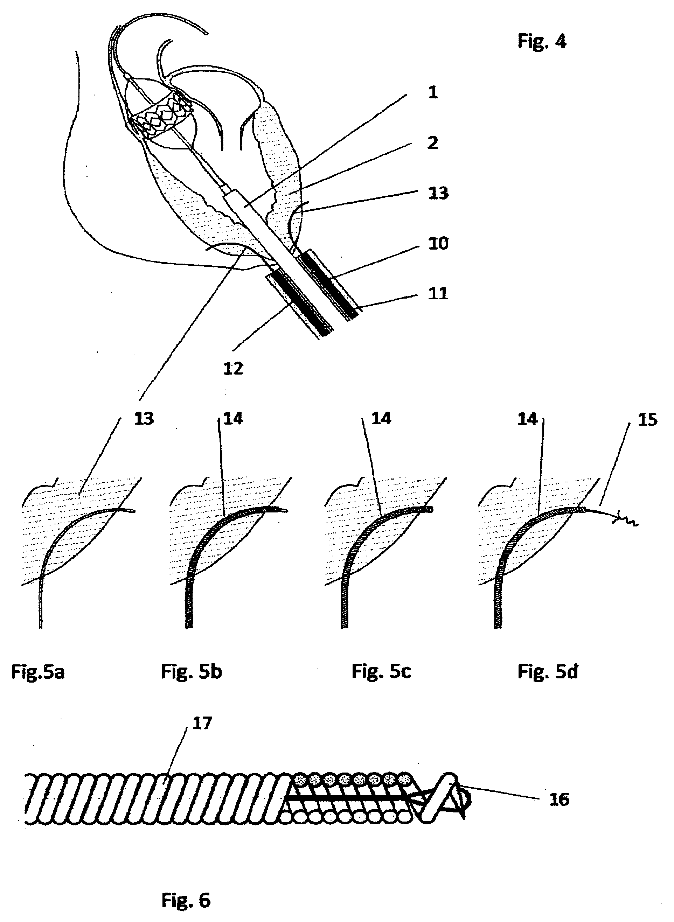

**Fig. 4** zeigt eine beispielhafte Ausführungsform des Verschlusssystems in der linken Ventrikel Spitze mit einer zusätzlichen Einführung von temporären Elektroden für eine mögliche externe Herzstimulation. Über den Trokar (1), der das Verschlusssystem in sich birgt, wird ein zusätzlicher Katheter (10) geschoben, der an den inneren gegenüberliegenden Seiten zwei Führungskanäle (Lumen) (11, 12) für die Einführung der temporären Elektroden aufweist. Zunächst werden durch die Lumen vorgebogene Nitinol Stiletts (13) eingeführt, die einen Kanal für spätere Elektroden herstellen.

**Fig. 5a-5d** zeigt den Ablauf der Einführung und Fixierung der temporären Elektroden für die Stimulation des Herzens.

Zunächst wird ein vorgebogenes Stilett (13) so weit eingeführt, dass die Spitze wieder aus dem Myokard herausragt. Danach wird über das Stilett eine Wendel (14) in der gleichen Weise vorgeschoben. Das Stilett wird entfernt und die temporäre Elektrode (15) durch das Lumen der Wendel (14) eingeführt. Die proximalen Elektrodenteile werden außerhalb des Körpers an einen Herzschrittmacher angeschlossen. Die nachfolgende Vorgehensweise ist exakt die gleiche wie bei der routinemäßigen Anwendung von Elektroden (Herzdrähten) nach einer offenen Herzoperation. Nach Abschluss der Behandlung können die Elektroden leicht durch Ziehen aus dem Körper entfernt werden.

**Fig. 6** zeigt eine weitere beispielhafte Ausführungsform für das Einführen der temporären Stimulationselektrode durch den Führungskanal (11). Hierbei wird die Elektrode (16), die distal eine Öse als Fixierung besitzt, mit Hilfe einer separaten Wendel (17) durch das Myokard an die Außenwand der Herzens transportiert.

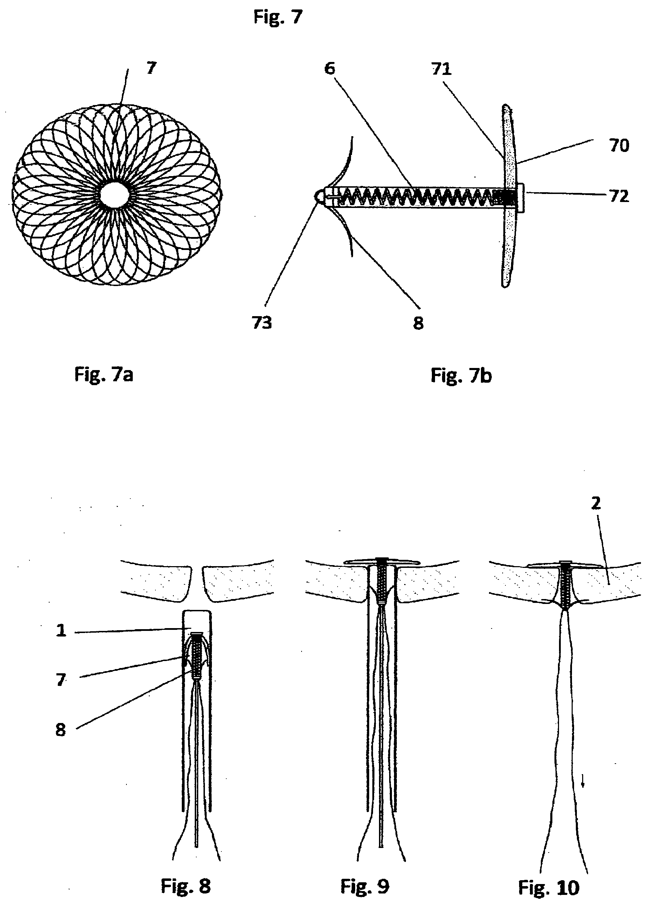

**Fig. 7a** **und b** zeigen die in Fig. 2 dargestellten Ausführungsformen im Detail.

**Fig. 7a** zeigt das Schirmgeflecht (7) aus Nitinoldraht in der Aufsicht. Man erkennt eine Vielzahl von feinen Fäden aus Nitinol. Beispielsweise besteht das Geflecht aus 80 Drähten.

**Fig. 7b** zeigt das erfindungsgemässe Verschlusssystem in Seitenansicht. Die Drahtenden laufen in der Fassung (72) zusammen. Hat sich das Schirmgeflecht entfaltet, so nimmt es die Form eines Regenschirms an. Die Membranbespannung erfolgt entweder an der Außenseite (70) oder an der Innenseite (71), vorzugsweise an der Außenseite (70). Es ist aber auch eine doppelte Membranbespannung der Außenseite und der Innenseite möglich. Das Schirmgeflecht befindet sich am Ende einer Schraubenfeder (6), die mit dem Fixierungselement (8) verbunden ist. Die Öse (73) dient zur Einführung des Doppelfadens (9).

**Die** **Fig. 8, 9 und 10** zeigen eine beispielhafte Applikationsprozedur des erfindungsgemäßen Verschlusssystems.

In **Fig**. **8** befindet sich das Verschlusssystem im Trokar (1). Das Schirmgeflecht (7) und das Fixierungselement (8), hier in Hakenform, sind zusammengefaltet. Der Stab dient zum Herausschieben des Verschlusssystems.

In **Fig. 9** hat der Trokar die Myokardöffnung gedehnt. Das Schirmgeflecht wird aus dem Trokar herausgeschoben und entfaltet sich in Form eines Regenschirms. Das Fixierungselement ist noch im Trokar und somit geschützt.

In **Fig. 10** wird der Zug am Faden (9) dargestellt. Durch Zug am Faden wird eine Vorspannung zwischen Myokard (2) und Schirmgeflecht (7) erzeugt. Das Fixierungselement wird unter Spannung unterhalb des Myokards gezogen. Nach Zurückziehen des Trokars bei gespannt gehaltenem Faden wird die Hakenstruktur (8) entspannt und dadurch im Myokardgewebe (2) verankert.

## Patentansprüche

1. Implantierbares Verschlusssystem zum Verschließen einer Öffnung im Myokard (2) der linken Herzkammerspitze bestehend aus:
a) einem elastischen Element (6) das ein distales und ein proximales Ende aufweist;
b) mindestens einem Schirmgeflecht (7) mit einer Außenseite und einer Innenseite, das sich entweder am distalen oder am proximalen Ende des elastischen Elements befindet;
c) einem Fixierungselement (8) das sich entweder am distalen oder am proximalen Ende des elastischen Elements (6) befindet und dem Schirmgeflecht (7) gegenüber angeordnet ist; wobei das Schirmgeflecht (7) aus Nitinoldraht besteht und mindestens 30 Fäden mit einem Durchmesser von 0.03-0,15mm aufweist und mindestens eine Seite des Schirmgeflechts eine Membranbespannung (70, 71) aufweist.

2. Verschlusssystem nach Anspruch 1, wobei das Schirmgeflecht (7) aus Nitinoldraht besteht und 30-100 Fäden mit einem Durchmesser von 0,05-0,13mm, besonders bevorzugt circa 0,1 mm aufweist.

3. Verschlusssystem nach Anspruch 1 oder 2, wobei die Membranbespannung (70, 71) aus einem biokompatiblen, flüssigkeitsdichten Kunststoff wie Silikon oder Polyurethan besteht.

4. Verschlusssystem nach einem der Ansprüche 1-3 wobei die Außenseite des Schirmgeflechts (70) bespannt ist.

5. Verschlusssystem nach einem der Ansprüche 1-4, wobei das elastische Element (6) eine Schraubenfeder ist.

6. Verschlusssystem nach einem der Ansprüche 1-5, wobei das Fixierungselement (8) Hakenstruktur aufweist.

7. Verschlusssystem nach einem der Ansprüche 1-6, wobei das Verschlusssystem zusammengefaltet ist und sich im Tubus eines Trokars (1) befindet.

## Claims

1. An implantable sealing device for sealing in the myocardium (2) a tissue opening of the left ventricular apex comprising:
a) an elastic member (6) having a distal and a proximal end;
b) at least one umbrella shaped braiding (7) having an outside face and an inside face and being positioned either at the distal end or at the proximal end of the elastic element;
c) a fixing member (8) located either at the distal end or at the proximal end of the elastic element (6) and placed opposite to the umbrella shaped braiding (7);
wherein the umbrella shaped braiding (7) consists of at least 30 threads of nitinol wire with a diameter between 0.03mm and 0.15mm and at least one face of the umbrella shaped braiding is coated by a membrane (70, 71).

2. The sealing device according to claim 1, wherein the umbrella shaped braiding (7) consists of 30-100 threads of nitinol wire with a diameter between 0.05mm and 0.13mm, preferably a diameter about 0.1mm.

3. The sealing device according to claim 1 or 2, wherein the membrane covering (70, 71) consists of a biocompatible and fluid tight plastic, such as silicone or polyurethane.

4. The sealing device according of any of claims 1 to 3, wherein the outside face (70) of the umbrella shaped braiding is coated by a membrane.

5. The sealing device according of any of claims 1 to 4, wherein the elastic member (6) is a coil spring.

6. The sealing device according of any of claims 1 to 5, wherein the fixing member (8) has a hook structure.

7. The sealing device according of any of claims 1 to 6, wherein the device is folded inside the tube of the trocar (1).

## Revendications

1. Un système d'obturation implantable pour obturer une ouverture dans le myocarde (2) de l'apex ventriculaire gauche comprenant :
a) Un élément élastique (6) présentant une extrémité distale et une extrémité proximale ;
b) Au moins une tresse en forme de parapluie (7) ayant un côté extérieur et un côté intérieur et qui est située soit à l'extrémité distale soit à l'extrémité proximale de l'élément élastique ;
c) Un élément de fixation (8) situé soit à l'extrémité distale soit à l'extrémité proximale de l'élément élastique (6) et disposé du côté opposé à la tresse en forme de parapluie (7) ; dans lequel la tresse en forme de parapluie (7) est constituée de fils de Nitinol et comprend au minimum 30 fils de diamètre compris entre 0,03 et 0,15mm, et dans lequel au moins un des côtés de la tresse en forme de parapluie est revêtu par une membrane (70, 71).

2. Un système d'obturation selon la revendication 1, dans lequel la tresse en forme de parapluie (7) est constituée de fils de Nitinol et comprend 30-100 fils de diamètre compris entre 0,05 et 0,13mm, de préférence de diamètre d'environ 0,1mm.

3. Un système d'obturation selon la revendication 1 ou 2, dans lequel la membrane (70, 71) de revêtement est réalisée en matière plastique biocompatible et étanche aux liquides, tel que le silicone ou le polyuréthane.

4. Un système d'obturation selon l'une quelconque des revendications 1 à 3, dans lequel le côté extérieur de la tresse en forme de parapluie (70) est revêtu par la membrane.

5. Un système d'obturation selon l'une quelconque des revendications 1 à 4, dans lequel l'élément élastique (6) est un ressort hélicoïdal.

6. Un système d'obturation selon l'une quelconque des revendications 1 à 5, dans lequel l'élément de fixation (8) a une structure de crochet.

7. Un système d'obturation selon l'une quelconque des revendications 1 à 6, dans lequel le système d'obturation replié sur lui-même est logé dans le tube du trocart (1).
